# FASCICULE DE BREVET EUROPEEN

(11) **EP 0 670 147 B1**
(45) Date de publication et mention de la délivrance du brevet: **29.08.2001**
(21) Numéro de dépôt: 95400392.7
(22) Date de dépôt: 24.02.1995
(51) Int. Cl.: A61B 19/00, G10K 9/13, B06B 1/02

(54) **Procédé et appareil de thérapie générant des ultrasons à effet de cavitation réduite**
Verfahren und Therapiegerät zur Erzeugung von Ultraschall mit reduzierter Kavitationsbildung
Therapeutical procedure and apparatus for generation of ultrasound with reduced cavitation

(30) Priorité: 01.03.1994 FR 9402322
(43) Date de publication de la demande: 06.09.1995
(73) Titulaire: TECHNOMED MEDICAL SYSTEMS, 69120 Vaux-en-Velin (FR); INSTITUT NATIONAL DE LA SANTE ET DE LA RECHERCHE MEDICALE (INSERM), 75654 Paris Cédex 13 (FR)
(72) Inventeur: Chapelon, Jean-Yves, F-69100 Villeurbanne (FR)
(74) Mandataire: Hirsch, Marc-Roger

(56) Documents cités:
- EP-A- 0 274 136
- DE-A- 2 524 555
- FR-A- 2 685 211
- US-A- 3 866 068
- PROCEEDINGS OF 1991 ULTRASONICS SYMPOSIUM, vol. 1, WALT DISNEY WORLD VILLAGE, LAKE BUENA VISTA, FLORIDA, USA, page 1357 J. Y. CHAPELON ET AL. 'Effects of cavitation in the high intensity therapeutic ultrasound'
- JOURNAL OF THE ACOUSTICAL SOCIETY OF AMERICA, vol. 83, NEW YORK US, page 2190 J. B. FOWLKES ET AL. 'Cavitation threshold measurements for microsecond long pulses of ultrasound.'

## Description

La présente invention concerne essentiellement un procédé et un appareil de thérapie, générant des ultrasons de haute densité à effet de cavitation contrôlé, ainsi qu'une utilisation de ce procédé et de cet appareil pour la réduction des lobes secondaires créés par une structure périodique.

On sait que la thérapie par ultrasons, à l'aide de transducteurs piézo-électrique activé par des signaux électronique de type sinusoïdal, permet de créer des lésions tissulaires par échauffement des tissus par absorption des ultrasons. En outre, ces lésions tissulaires peuvent être réalisées dans un volume limité en réalisant la thérapie avec des ultrasons focalisés, ce qui est particulièrement intéressant pour un traitement efficace dans le cadre des thérapies du cancer comme, par exemple, de la prostate, du sein, du cerveau, etc.

Les appareils d'hyperthermie par ultrasons existants chauffent les tumeurs à une température modérée de l'ordre de 42,5°C, pendant des temps de l'ordre d'une heure.

Etant donné qu'un traitement par hyperthermie peut être insuffisant, il peut être avantageux de chercher à obtenir une température beaucoup plus élevée, par exemple d'environ 80°C, en vue d'une sensibilisation ou d'une destruction complète des cellules. Pour cela, il faut délivrer l'énergie acoustique au tissu dans un temps court, en général de l'ordre de quelques secondes, afin d'éviter notamment des déperditions de chaleur par la perfusion naturelle notamment due à la circulation sanguine, dans le tissu. L'énergie doit être suffisante, ce qui implique d'utiliser des intensités ultrasonores élevées.

Mais on se heurte alors aux problèmes techniques résultants des phénomènes de cavitation qui sont d'autant plus accentués que l'intensité acoustique est forte comme cela est décrit en détail par l'article de K. Hynynen publié dans la revue Ultrasound In Medecine and Biology, vol. 17, n°2, pages 157-171, (1991), ayant pour titre "The threshold for thermally significant cavitation in dog's thigh muscle in vivo".

On appelle cavitation acoustique tout phénomène physique impliquant l'activité de bulles ou microbulles gazeuses mises en mouvement par un champ acoustique.

On distingue en général deux types de cavitation:
- la cavitation stable pour laquelle les parois des bulles oscillent à la fréquence du champ ultrasonore sans trop de conséquence sur les cellules environnantes, mais par contre qui perturbe fortement la transmission ultrasonore en réfléchissant ou en diffusant les ondes incidentes. Ce phénomène peut apparaître pour des niveaux de pression très bas dès lors que des bulles sont présentes dans le milieu,
- la cavitation transitoire pour laquelle les bulles grossissent jusqu'à leur taille de résonnance avant d'imploser violemment. Dans ce cas, l'énergie accumulée par les bulles est libérée à la fois sous forme d'onde de choc, de chaleur intense (généralement de 1000 à 20 000 K) et de microjets atteignant des vitesses de 100 m/s. Tout ceci conduit à la création de radicaux libres et à la destruction mécanique des tissus environnants. Généralement, ce phénomène apparaît à partir de pressions incidentes élevées qui définissent ainsi le seuil de cavitation.

Tout milieu vivant contient une certaine quantité de gaz dissous présent sous forme de microgermes de bulles. Sous l'action d'un champ ultrasonore, ces microgermes vont grossir par un phénomène physique appelé diffusion redressée ("rectified diffusion") jusqu'à une taille critique appelée seuil de Blake.

Il y a quelques temps, il a été montré dans un article réalisé par l'inventeur intitulé "Effects of cavitation in the high intensity therapeutic ultrasound" publié aux pages 1357 à 1360 du volume 2 de 1991 de la revue Ultrasonics Symposium Proceedings (éditeur B.R. McAvoy) que l'utilisation d'intensités trop élevées, en général supérieures à 3000 W/cm², réduisait les effets thérapeutiques du traitement thermique impliquant une destruction des tissus. Pour expliquer ce phénomène, il est supposé qu'à ces intensités les bulles de cavitation qui pourraient apparaître en avant de la tache focale faisaient office d'écran aux ondes ultrasonores incidentes. Par ailleurs, c'est dans l'optique de réduire les effets de cavitation que F.J. Fry stipule dans le document WO-A-89/07909, qu'il est nécessaire d'inhiber la production de microbulles dans le site focal primaire pour éviter l'apparition de lésions en dehors de ce site (voir page 15 de ce brevet). Dans ces conditions, il est mentionné que l'intensité ne soit pas dépasser 300 W/cm² à la fréquence de 1 MHz ou 2100 W/cm² à 4 MHz.

K. Hynynen a également montré dans l'article précité qu'une intensité de 700 W/cm²/MHz doit être la valeur maximale à utiliser dans les traitements d'hyperthermie puisqu'à des niveaux supérieurs la cavitation induit une absorption d'énergie imprévisible.

En résumé de l'état de la technique antérieure, la cavitation gêne la pénétration des ondes acoustiques dans le tissu et empêche donc que le traitement se fasse de manière prévisible. D'autre part, la cavitation peut provoquer une destruction incontrôlée du tissu, en dehors du volume visé. Il y a donc lieu, quelle que soit l'application envisagée (c'est-à-dire un traitement thermique à température élevée détruisant les tissus, ou à une température modérée ou hyperthermie), d'augmenter les seuils de cavitation.

Pour éviter la cavitation, les seules recommandations que l'on peut trouver dans l'art antérieur consistent soit à réduire l'intensité acoustique, soit à émettre d'une façon discontinue, par trains d'ondes de forte durée déterminée, en respectant un temps d'attente entre les trains, soit enfin d'augmenter la fréquence d'émission.

Or, la réduction de l'intensité acoustique ou une émission discontinue conduisent à réduire l'énergie acoustique transmise au milieu, ce qui limite l'élévation de température ou augmente les temps de traitement. Enfin, l'augmentation de la fréquence d'émission limite la profondeur de traitement, l'absorption dans les tissus étant directement proportionnelle à la fréquence comme cela est décrit par Daniels et al. dans la revue Ultrasound in Medecine and Biology, vol. 13, n°9, (1987) avec l'article intitulé "Ultrasonicaly induced gas bubble production in agar based gels". Il est à noter également que dans l'art antérieur, pour chauffer les tissus, il est utilisé des ondes sinusoïdales continues, c'est-à-dire que la durée d'émission est très supérieure à la période du signal. Habituellement, il est préconisé d'insonifier le tissu pendant plusieurs secondes à une fréquence comprise entre 1 et 5 MHz.

Certains auteurs ont préconisé au contraire d'utiliser des ondes acoustiques impulsionnelles, de durée de l'ordre de quelques périodes du signal, soit environ quelques microsecondes mais dans un but totalement différent, à savoir soit pour la destruction de concrétions (lithotritie), soit pour le diagnostic (échographie et Doppler).

Les phénomènes de cavitation produits par ces impulsions ont été étudiés. Par exemple, Fowlkes and Crum dans l'article ayant pour titre "cavitation threshold measurements for microsecond length pulses of ultrasound" paru dans J. Acoustic Soc. Am., 83, (6), Juin 1988, a étudié l'évolution du seuil de cavitation en fonction de la largeur des impulsions et de la fréquence des impulsions. De même Delius, en étudiant la cavitation produite par des lithotriteurs, recommande de réduire le taux de répétition des ondes acoustiques ("Effects of lithotripter shock waves on tissue and materials", Frontiers on non-linear acoustics, édité par MF Hamilton et DT Blackstock, ElSevier Science Publishers, Londres, 1990).

Or, les méthodes impulsionnelles ne permettent pas de produire une élévation de température dans le tissu puisque chaque impulsion ne transporte que peu d'énergie et qu'il faut espacer ces impulsions. On ne peut donc pas assimiler les travaux faits avec ces ondes avec les travaux à la base de la présente invention.

Ainsi, la présente invention a pour but principal de résoudre le nouveau problème technique consistant en la fourniture d'une solution permettant de délivrer le maximum d'énergie au milieu, de préférence des tissus d'un être vivant, en particulier un animal ou un être humain, dans le temps le plus court possible de préférence en réduisant ou en empêchant les phénomènes de cavitation.

La présente invention a encore pour but de résoudre le nouveau problème technique consistant en la fourniture d'une solution permettant de délivrer le maximum d'énergie au milieu, de préférence des tissus d'un être vivant, en particulier un animal ou un être humain, dans le temps le plus court possible, tout en permettant un contrôle efficace et sûr du dépôt de chaleur, en permettant ainsi de réaliser indifféremment un traitement thermique modéré comme dans le cadre de l'hyperthermie, ou un traitement thermique à température élevée, pour réaliser une destruction des tissus, de préférence en réduisant ou empêchant les phénomènes de cavitation.

Par ailleurs, l'invention résout le problème nouveau des focalisations secondaires qui peuvent se produire lorsque des structures périodiques ou quasi-périodiques sont interposées entre le dispositif d'émission et la zone à traiter.

La présente invention a encore pour but de résoudre le nouveau problème technique énoncé ci-dessus d'une manière simple, fiable, peu coûteuse, permettant une large diffusion industrielle et médicale.

La présente invention apporte pour la première fois une solution satisfaisante à ces problèmes techniques ainsi définis et présente d'autres avantages techniques qui apparaîtront clairement à l'homme de l'art à partir de la description détaillée qui va suivre de l'invention, incluant les figures annexées qui en constituent une partie intégrante.

Ainsi, selon un premier aspect, la présente invention fournit un procédé de génération d'ondes ultrasonores dans un milieu de propagation comprenant l'activation d'au moins un élément transducteur ultrasonore par un signal électronique délivré par un générateur de signal, caractérisé en ce qu'on réduit ou empêche les phénomènes de cavitation résultant de la propagation des ondes ultrasonores émises par l'élément de transducteur ultrasonore dans le milieu de propagation en utilisant un générateur de signal délivrant un signal électronique activant l'élément transducteur pour émettre un signal acoustique large bande dont la largeur de bande vaut environ 50% de la fréquence centrale du générateur de signal.

Dans le cadre de l'invention, c'est-à-dire de la description et des revendications, on entend par spectre large bande d'un signal électronique le fait que la largeur de bande du spectre de ce signal vaut environ 50% de la fréquence centrale. Par exemple, pour un signal à fréquence centrale à MHz, la largeur de bande sera d'environ 1 MHz et comprendra donc des fréquences variant d'environ 1,5 MHz à environ 2,5 MHz.

Selon un mode de réalisation avantageux de ce procédé, on utilise un générateur de signal délivrant un signal électronique aléatoire ou pseudo-aléatoire continu.

Dans le cadre de l'invention, c'est-à-dire de la description et des revendications, on entend par signal continu le fait que la durée d'émission du signal est très supérieure à la période du signal, comme cela a aussi été décrit dans la partie introductive de la présente description.

Selon un mode de réalisation avantageux de l'invention, le générateur de signal précité délivre un signal électronique pseudo-aléatoire de type Gaussien ou Poissonien. Ces signaux peuvent être, par exemple, obtenus par une source de bruit thermique amplifié par un amplificateur électronique.

Selon un autre mode de réalisation avantageux, le générateur de signal précité délivre un signal électronique pseudo-aléatoire de Golay.

Selon encore un autre mode de réalisation avantageux, le générateur de signal précité délivre un signal électronique pseudo-aléatoire codé de Barker.

Selon encore un autre mode de réalisation de l'invention, le générateur de signal précité délivre un signal électronique codé de type pseudo-aléatoire de séquence M.

Les signaux à séquence "M" ou encore dénommés séquences binaires à longueur maximale sont du type de ceux qui sont décrits au chapitre 6 par Jean-Yves CHAPELON, pages 225 à 236, en particulier à partir de la page 230 du livre "Progress in medical imaging", édité par le Professeur Newhouse, éditeur Springer Verlag, New York, année 1988.

Ces signaux pseudo-aléatoires codés de séquence M, ou de "Golay" ou de "Barker" peuvent être utilisés directement ou peuvent moduler en phase ou en fréquence un signal électronique dont la fréquence porteuse correspond à la fréquence nominale de fonctionnement du transducteur.

On préfère en particulier les signaux codés de type pseudo-aléatoire de séquence "M". Ces signaux sont décrits précisément dans "Progress in medical imaging". Il s'agit succinctement de séquences de signaux binaires construits par la répétition pseudo-aléatoire d'impulsions ou "pulses" d'une durée élémentaire. Chacune de ces séquences se répète avec une période de répétition "T" caractéristique de la séquence "M".

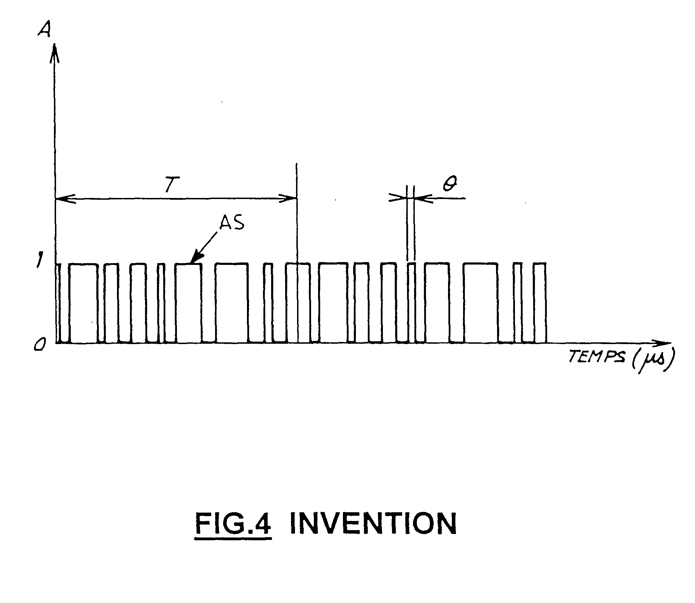

On peut donner une description plus précise d'un signal de séquence "M" en référence à la figure 4 annexée:
- durée d'impulsion ou "pulse" élémentaire "θ":
   0,1 s < 100 *µ*s, idéalement d'environ 1 *µ*s,
- période de répétition "T": 1 *µ*s < T < 10 s, idéalement comprise entre 0,5 et 5 s.

Les signaux codés de type pseudo-aléatoire, en particulier de type pseudo-aléatoire de séquence "M" actuellement préférés, sont réalisables aisément à partir de circuits électroniques bien connus de l'homme de l'art.

Grâce à ces signaux électroniques larges bandes, de préférence aléatoires ou pseudo-aléatoires, on contrôle mieux le dépôt de chaleur et on évite que l'augmentation de température ne soit perturbée par des effets secondaires comme la cavitation, ce qui permet d'éviter une destruction tissulaire spontanée, en particulier dans le cas d'un chauffage modéré comme dans le cadre d'une hyperthermie.

D'autre part, dans le cadre d'utilisation d'intensité élevée, l'invention permet d'utiliser des intensités plus élevées et de réduire, pour une même dose ultrasonore, la durée des tirs et par conséquent la durée du traitement, tout en évitant les phénomènes de cavitation, ce qui permet de réaliser des traitements de tumeur d'être vivants, en particulier des animaux ou des êtres humains, avec une sécurité accrue et en réduisant les risques de dommages au niveau des interfaces.

Selon un deuxième aspect, la présente invention fournit également un appareil de thérapie comprenant un dispositif de thérapie proprement dit comprenant au moins un élément transducteur ultrasonore de thérapie et un générateur de signal délivrant un signal électronique audit élément transducteur ultrasonore, caractérisé en ce que le générateur de signal délivre un signal électronique aléatoire ou pseudo-alétoire activant l'élément transducteur pour émettre un signal acoustique large bande, dont la largeur de bande vaut environ 50% de la fréquence centrale du générateur de signal.

Selon un mode de réalisation avantageux de l'appareil, le générateur de signal délivre un signal aléatoire de type Gaussien ou Poissonien.

Selon un autre mode de réalisation avantageux, le générateur de signal délivre un signal pseudo-aléatoire codé de Golay ou un signal pseudo-aléatoire codé de Barker.

Selon encore un autre mode de réalisation avantageux, le générateur de signal délivre un signal pseudo-aléatoire codé à séquence M.

Ce signal pseudo-aléatoire codé à séquence M présente de préférence une fréquence présentant une durée d'impulsion élémentaire thêta (θ) comprise entre 0,1 *µ*s et 100 *µ*s, idéalement d'environ 1 *µ*s, et une période de répétition T comprise entre 1 *µ*s et 10 s, idéalement comprise entre 0,5 et 5 s.

Dans l'un ou l'autre des aspects de l'invention, pour améliorer l'efficacité de la réduction ou de l'empêchent des effets de cavitation, on utilisera de préférence un transducteur ultrasonore lui-même à large bande, c'est-à-dire que ce transducteur ultrasonore génère des ondes ultrasonores lorsqu'il est excité par un signal, dont la fréquence est sensiblement différente de sa fréquence nominale.

Habituellement, on peut utiliser deux types de transducteur pour la génération d'ondes ultrasonores continues à but thérapeutique. Il s'agit d'une part des transducteurs ultrasonores classiques qui sont essentiellement formés d'une céramique piézoélectrique. Ces céramiques ont une impédance acoustique très différente de celle du milieu de propagation et en conséquence leur couplage avec ce milieu est faible. Il en résulte que la céramique vibre fortement lorsqu'elle est excitée par un signal électrique et que le transducteur ultrasonore résultant est à bande de fréquence étroite.

Pour la thérapie antérieurement réalisée avec des ondes ultrasonores, ces transducteurs sont appropriés dans la mesure où les signaux de contrôle sont eux-mêmes à bande étroite.

Dans le cadre de l'invention, on élargira la bande de fréquence des transducteurs ultrasonores en appliquant avantageusement à la surface de la céramique différents types de traitements qui modifient le couplage ultrasonore avec le milieu de propagation. En particulier, on déposera dans ce cas sur la céramique une couche de matériaux d'impédance acoustique intermédiaire entre la céramique et le milieu de propagation et d'épaisseur appropriée, cette couche étant appelée "couche quart-d'onde". On pourra ainsi déposer à l'arrière de la céramique, entre celle-ci et l'air, un revétement que l'on appelle "backing". Ces deux types de traitement de la céramique conduisent à réaliser un transducteur ultrasonore à large bande.

Un autre type de transducteur à large bande peut être réalisé en matériaux de type composite et qui et particulièrement adapté à la mise en oeuvre de la présente invention pour la réduction ou l'empêchement des effets de cavitation.

On obtient ainsi les avantages techniques déterminant précédemment énoncés.

Selon un troisième aspect, la présente invention propose un procédé de génération d'ondes ultrasonores dans un milieu de propagation, comprenant l'activation d'au moins un élément transducteur ultrasonore par un signal électrique périodique délivré par un générateur de signal, caractérisé en ce que l'on réduit ou l'on empêché les phénomènes de focalisation secondaire derrière une structure périodique ou quasi-périodique en utilisant un générateur de signal délivrant un signal électronique activant l'élément transducteur pour émettre un signal acoustique large bande, dont la largeur de bande vaut environ 50% de la fréquence centrale du générateur de signal. Ce procédé permet de réduire ou empêcher les phénomènes de focalisation secondaire derrière une structure périodique ou quasi-périodique.

Ceci permet par exemple de traiter le foie derrière le gril costal en réduisant ou empêchant les lésions dues à des phénomènes de focalisation secondaire ou lobes secondaires.

Dans un mode de mise en oeuvre de ce troisième aspect de l'invention, ledit signal électronique large bande est un signal de type aléatoire ou pseudo-aléatoire.

Le signal électronique large bande peut être un signal aléatoire de type Gaussien ou Poissonien.

Le signal électronique large bande peut aussi être un signal pseudo-aléatoire codé de Barker ou codé de Golay.

On peut, pour le signal électronique large bande, utiliser un signal pseudo-aléatoire codé à séquence M, de fréquence présentant une durée d'impulsion élémentaire thêta comprise entre 0,1 µs et 100 µs, idéalement d'environ 1 µs, et une période de répétition T compris entre 1 *µ*s et 10 s, idéalement comprise entre 0,5 et 5 s.

Le signal électronique large bande peut présenter une fonction d'autocorrélation s'approchant d'un Dirac.

D'autres caractéristiques apparaîtront également à l'homme de l'art à partir de la description suivante, incorporant les dessins qui en font partie intégrante, ainsi que des revendications qui font également partie de la présente description.

L'invention va maintenant être décrite avec un mode de réalisation actuellement préféré donné simplement à titre d'illustration et qui ne saurait donc en aucune façon limiter la portée de l'invention, en référence aux dessins annexés, dans lesquels:
- la figure 1 représente un schéma de principe général d'un appareil de thérapie connu pour réaliser une thérapie des tissus d'un être vivant, comprenant un dispositif mono ou multitransducteur ayant un ou des transducteurs piézoélectriques, en forme de coupelle sphérique, permettant une focalisation géométrique dans l'axe de propagation comme visible à la figure 1;
- la figure 2 représente la courbe formée par une onde sinusoïdale en fonction du temps t en abscisse avec l'amplitude A en ordonnée, selon l'art antérieur telle qu'utilisée dans le cadre du dispositif générateur d'ondes ultrasonores, conforme à la figure 1;

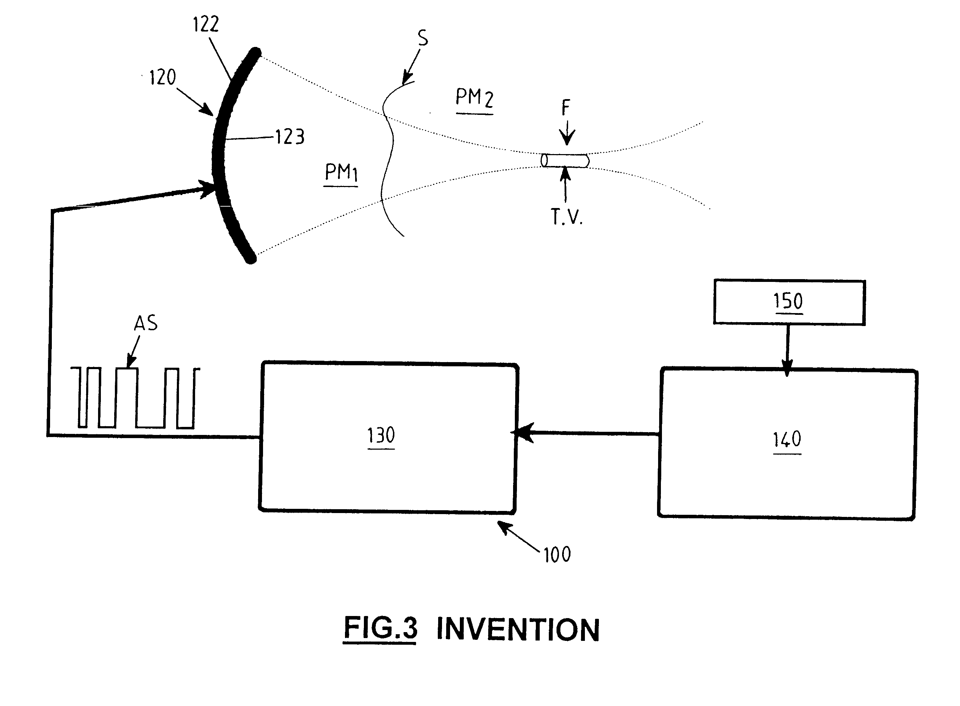

- la figure 3 représente un schéma de principe général d'un appareil de thérapie selon la présente invention pour réaliser une thérapie des tissus d'un être vivant, comprenant un dispositif de génération d'un signal électronique large bande, de préférence aléatoire ou pseudo-aléatoire transmis au dispositif transducteur piézoélectrique pour la génération d'une onde ultrasonore, l'onde du signal aléatoire ou pseudo-aléatoire étant représentée schématiquement à la figure 3 et en détail à la figure 4 en fonction du temps exprimé en microsecondes en abscisse, et de l'amplitude exprimée en ordonnées.
- la figure 5 représente un schéma de principe d'une installation permettant de mettre en évidence la réduction de la cavitation en utilisant un signal électronique de type aléatoire ou pseudo-aléatoire pour exciter le transducteur ultrasonique;

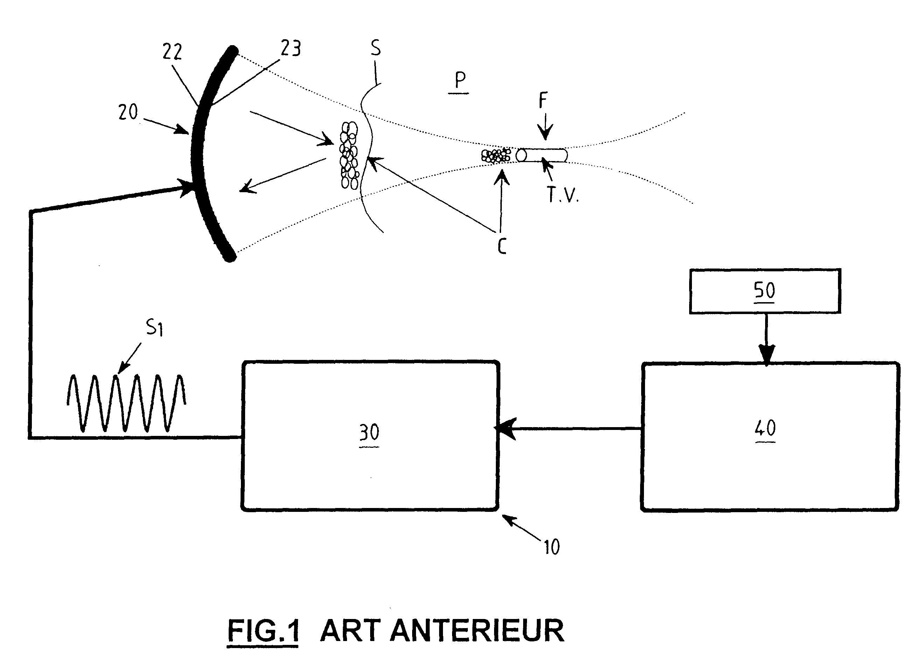

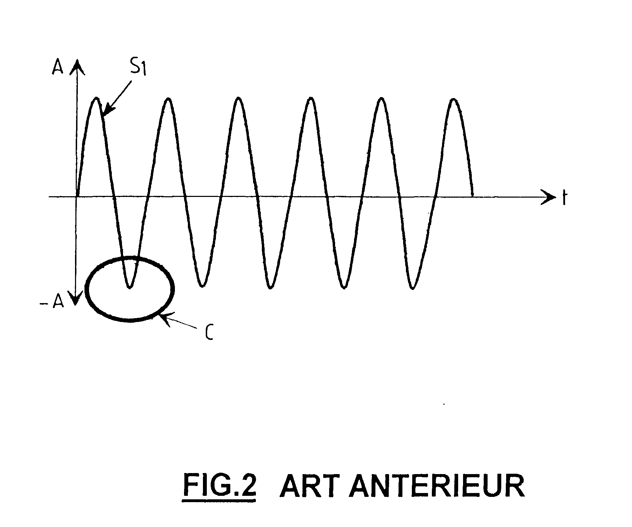

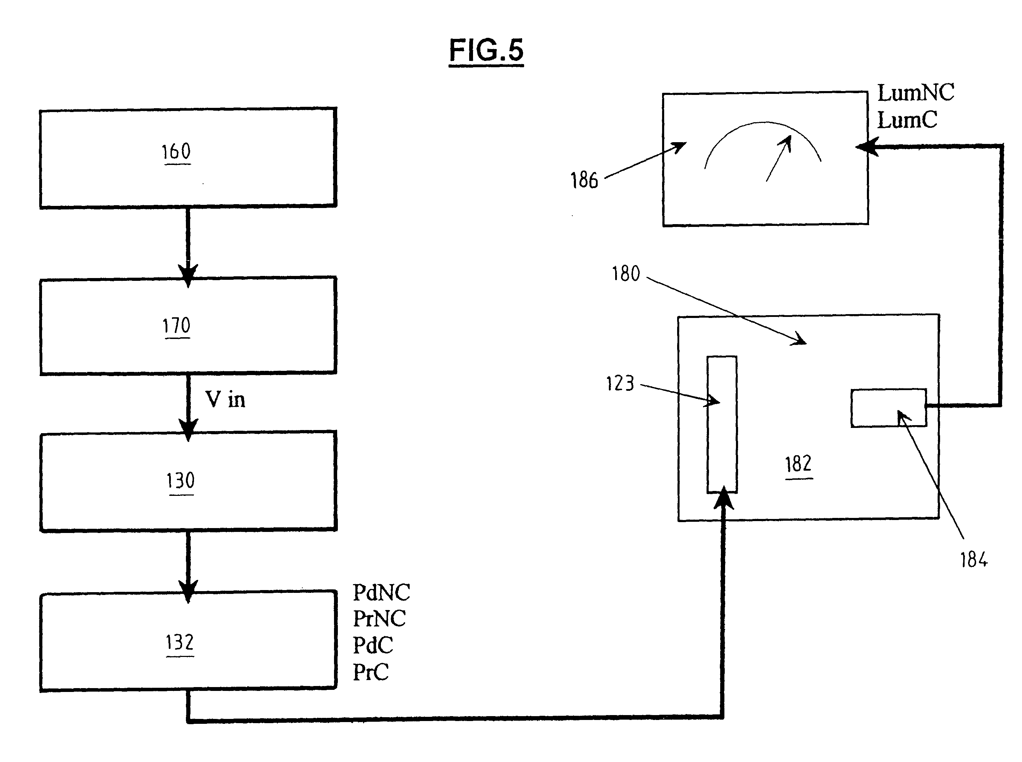

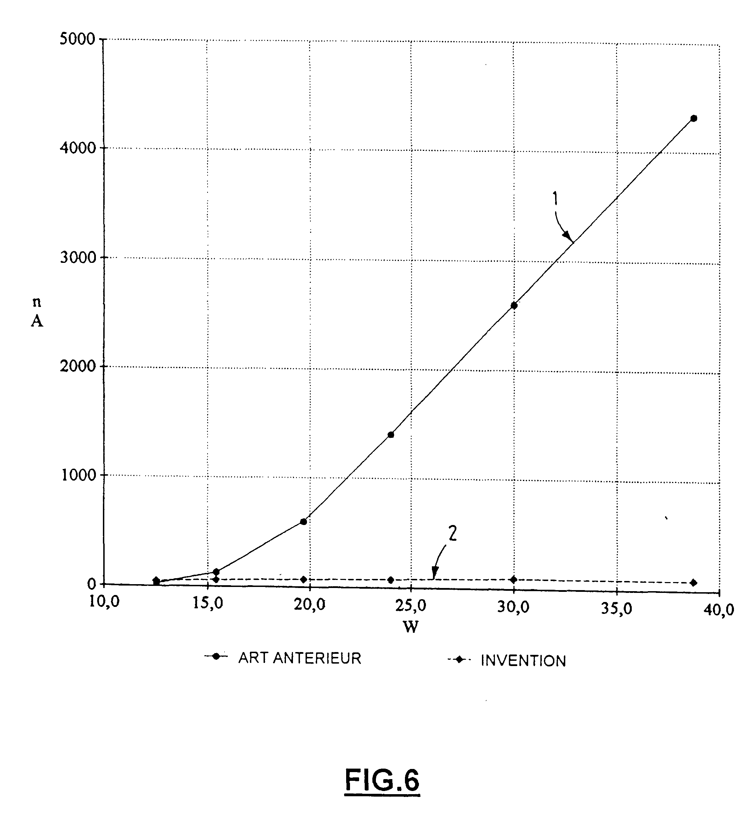

- la figure 6 représente les résultats obtenus avec l'appareillage de la figure 5 sous forme de courbe, respectivement la courbe référencée 2 avec le signal aléatoire ou pseudo-aléatoire selon la présente invention des figures 3 et 4 représentées par les signes - ◆ -, et la courbe référencée 1 avec le signal sinusoïdal selon l'art antérieur du type utilisé dans les figures 1 et 2, représentée par les signes - ● -, en fonction de la puissance transmise par le transducteur, exprimée en Watt en abscisse et avec en ordonnée la valeur en nano-ampère donnée par un ampèremètre intégrant la quantité totale de lumière émise par le luminol utilisé;
- la figure 7 représente un schéma de principe du fonctionnement d'un dispositif de l'art antérieur, en présence d'une structure périodique ou quasi-périodique;
- la figure 8 représente un schéma analogue à celui de la figure 7, dans un dispositif mettant en oeuvre l'invention.

En référence à la figure 1, on a représenté schématiquement un appareil de thérapie connu représenté par le mode de référence général 10, pour réaliser la thérapie de tissus d'un être vivant.

Cet appareil de thérapie 10 comprend un dispositif de thérapie proprement dit ici sous forme de calotte sphérique 22 à focalisation naturelle comprenant un ou plusieurs éléments transducteurs piézoélectriques, en particulier un monoélément transducteur piézoélectrique référence 23 ayant sensiblement la même dimension que la calotte sphérique 22, comme cela est bien connu à l'homme de l'art et aucune description complémentaire n'apparaîtra nécessaire. Un exemple de réalisation d'un tel dispositif de thérapie est, par exemple, sous forme de calotte sphérique 22 de diamètre 100 mm à focalisation naturelle à la distance focale de 100 mm, ayant une fréquence de fonctionnement de 1 MHz environ, réalisé avec un seul élément transducteur piézoélectrique 23.

L'élément transducteur 23 est relié via un dispositif amplificateur 30 à un générateur de signal 40 qui peut être lui-même commandé par une centrale de commande 50.

On notera que, selon un autre mode de réalisation possible, la calotte sphérique 22 à focalisation naturelle peut être subdivisée en réseau annulaire, bien connu à l'homme de l'art, ou en mosaïque, également bien connu à l'homme de l'art, et aucune description complémentaire n'apparaît nécessaire. Dans ce cas, chaque élément transducteur annulaire ou en mosaïque est relié via un dispositif amplificateur comprenant des amplificateurs individuels et un dispositif de lignes à retard comprenant des lignes individuelles à retard, à un générateur de signaux communs tel que le générateur de signal 40 lui-même commandé par une centrale de commande telle que la centrale 50. La centrale de commande commande alors les lignes à retard en fournissant la valeur de retard nécessaire à chaque ligne pour obtenir une focalisation à la distance de focalisation recherchée.

Grâce à cette conception de l'appareil, il est possible de réaliser une focalisation dynamique électronique avec variation à volonté de la distance focale.

Dans le cadre de l'appareil de l'art antérieur représenté à la figure 1, lorsqu'on fait générer par le générateur 40 un signal électronique classique de type sinusoïdal, comme représenté schématiquement à la sortie de l'amplificateur 30 et de manière plus détaillée à la figure 2, par exemple une fréquence d'environ 1 MHz, on obtient une focalisation naturelle dans la zone focale F définissant le volume de traitement T.V.

Avec un tel signal sinusoïdal continu, c'est-à-dire présentant une durée d'émission nettement supérieure à la valeur de la période, en général de plusieurs secondes par rapport à une fréquence de 1 MHz (période 1 *µ*s), les bulles de gaz présentes dans les tissus, ou générées spontanément par les ondes ultrasonores, référencées B, présentes à l'avant des divers interfaces, par exemple de la zone focale F ou de l'interface avec la peau S du patient P, vont osciller au bout de quelques pulsations à la fréquence fₚ, appelée fréquence de pompage. Le diamètre de ces bulles de gaz B va augmenter à chaque pulsation selon le phénomène de diffusion rectifiée, en anglais "rectified diffusion", pour atteindre une valeur maximale caractéristique de la fréquence fₚ ou seuil de Blake. Lorsque l'intensité du champ acoustique dépasse le seuil de cavitation, ce qui est pratiquement toujours le cas dans le cas de traitement thérapeutique, la bulle implose en libérant de l'énergie. Ce seuil de cavitation est représenté par la référence générale C à la figure 2 pour des intensités du signal sinusoïdal ayant une amplitude relativement importante.

Ceci constitue un inconvénient majeur de l'état de la technique antérieure.

Dans ces conditions, et en référence à la figure 3, on a représenté un appareil selon la présente invention, représenté par le numéro de référence générale 100. Pour les pièces identiques ou de fonction identique à celles de l'appareil de l'art antérieur, on utilisera le même numéro de référence mais simplement augmenté de 100. Ainsi, le dispositif de thérapie proprement dit est référencé 120, la calotte sphérique 122 et l'élément transducteur piézoélectrique 123, le dispositif amplificateur 130, le dispositif générateur de signal électronique 140 et la centrale de commande 150.

Dans le cadre de l'appareil selon l'invention, le dispositif générateur de signal 150 délivre un signal électronique large bande appelé A.S. représenté à la figure 3 à la sortie du dispositif amplificateur 130, et de manière plus détaillée à la figure 4.

Dans le cadre de l'invention, le transducteur ultra-sonore est lui-même large bande en étant réalisé comme précédemment décrit.

Dans le cadre de l'invention et pour la réalisation d'une sonde endorectale pour le traitement de la prostate, on préfère utiliser un transducteur en forme de calotte sphérique d'un diamètre d'environ 35 mm à focalisation naturelle à la distance focale de 35 mm, tandis qu'on utilisera de préférence un signal à fréquence centrale d'environ 2,25 MHz.

Comme il a été précédemment indiqué, on entend par spectre large bande dans le cas de la présente description et des revendications, le fait que la largeur de bande du spectre de ce signal vaut environ 50% de la fréquence centrale. Par exemple, pour un signal à fréquence centrale à 2 MHz, la largeur de bande sera d'environ 1 MHz et comprendra donc des fréquences variant d'environ 1,5 MHz à environ 2,5 MHz.

Dans le cadre de l'invention, et plus particulièrement dans le but de réduire ou empêcher les phénomènes de cavitation résultant de la propagation des ondes ultrasonores émises par l'élément transducteur ultrasonore 123 dans le milieu de propagation PM1, PM2, le générateur de signal 140 délivre un signal électronique large bande aléatoire ou pseudo-aléatoire, référencé A.S. à la figure 3 et à la figure 4. La fréquence centrale est généralement comprise entre 20 MHz et 0,5 MHz.

On observera que dans le cadre de l'invention, l'utilisation de signaux aléatoires ou pseudo-aléatoires fournit un spectre du champ ultrasonore d'excitation qui est un spectre à fréquences multiples dont le caractère aléatoire d'apparition limite la croissance des bulles, de telle sorte qu'elles atteignent rarement les diamètres critiques à partir desquels se produisent les effets de cavitation.

Ainsi, l'invention permet de limiter ou d'empêcher le phénomène de cavitation.

Le signal aléatoire ou pseudo-aléatoire délivré par le générateur de signal 140 est du type précédemment décrit et peut être, par exemple, un signal électronique pseudo-aléatoire codé de Golay ou de Barker qui sont bien connus à l'homme de l'art, en particulier à partir de la description précédente.

Il peut également s'agir d'un signal électronique codé de type pseudo-aléatoire de séquence M, de préférence ayant une durée d'impulsion élémentaire thêta comprise entre 0,1 *µ*s et 100 *µ*s, une période de répétition T comprise entre 1 *µ*s et 10 s.

Il peut également s'agir d'un signal aléatoire de type Gaussien ou Poissonien.

Il est à noter que le signal électronique représenté à la figure 3 et en détail à la figure 4 est un signal électronique pseudo-aléatoire à séquence M dont la période T est comprise entre 1 *µ*s et 10 s et la durée d'impulsion élémentaire thêta (θ) est comprise entre 0,1 *µ*s et 100 *µ*s.

En référence à la figure 5, on a représenté un appareillage qui permet de mettre en évidence la réduction de la cavitation lorsqu'on utilise les signaux électroniques ou codes pseudo-aléatoires selon la présente invention, grâce à l'emploi du luminol. Ce composé chimique est connu pour émettre des photons (fluorescence) lorsqu'il est en présence de cavitation comme décrit dans l'article de FOWLKES et al. dans la revue J. Acoust. Soc. Am. Vol. 83, de Juin 1988, pages 2190-2200, ayant pour titre "Cavitation threshold measurements for microsecond lenght pulses of ultrasounds".

Cet appareil comprend schématiquement en référence à la figure 5, un générateur de fréquence 160 associé à un générateur de code 170, l'ensemble de ces deux générateurs 160 et 170 correspondent au générateur de signal électronique 140 de la figure 3, couplé à un dispositif amplificateur 130 pour délivrer un signal électronique de type pseudo-aléatoire ou aléatoire selon la présente invention, par exemple de séquence M, à l'élément transducteur 123 placé dans une cuve 180 contenant une solution de luminol 182. Dans la solution de luminol 182, non seulement le transducteur 123 est immergé, mais également un dispositif photomultiplicateur 184 relié lui-même à un nanoampèremètre à aiguille 186 permettant de donner une mesure en nanoampère proportionnelle à la photoluminescence obtenue grâce au luminol.

La solution de luminol 182 est obtenue à partir d'une solution d'eau distillée déminéralisée et saturée en air à pression atmosphérique, et du luminol (aminophtalhydrazide, 1,4 mmole dissous préalablement dans du diméthylsulfoxyde ou DMSO grade hplc). La solution est tamponnée avec du CAPS (acide 3-cyclohexylamino-1-propane sulfonique), 25 mmoles et équilibrée à pH 10 avec de la soude 0,1 M.

Pour cette expérience, l'élément transducteur ultrasonore 123 utilisé présente un diamètre de 5 cm, et focalise à 97 mm. La cuve est ici une cuve de 40 ml cylindrique disposée horizontalement, présentant une longueur de 5 cm dans laquelle l'effet de focalisation n'intervient pas, de sorte que l'énergie est répartie dans toute la cuve. La fréquence de résonnance du transducteur est de 1,11 MHz, valeur obtenue au maximum de sonoluminescence de l'eau.

Le photomultiplicateur est, par exemple, de type R374 Hamamatsu qui comprend un système optique de lentilles pour recevoir la fluorescence émise par le luminol. La cuve 180 est isolée optiquement pour éviter toute interférence lumineuse. Le photomultiplicateur 184 est alimenté par un petit générateur non représenté ici et le signal lumineux est lu sur l'ampèremètre 186 qui intègre la quantité totale de lumière émise par le luminol.

Le transducteur 123 est connecté à l'amplificateur de puissance 130, par exemple de type Ampar 801, Prana, qui présente une bande passante allant de 0,1 à 1,8 MHz. Le générateur de signal électronique ou code pseudo-aléatoire de séquence M est de fabrication INSERM, ayant une période T d'environ 60 ms, et une durée d'impulsion élémentaire thêta de 1,8 *µ*s et synchronisé par un générateur de fonction de type HP 8116 A. Le wattmètre 132 est de type digital de chez Rhode & Schwarz référence NAP et permet de contrôler les puissances incidente et réfléchie.

Pour ce wattmètre 132, les références indiquées à la figure 5 ont les significations suivantes:
"PdNC" puissance directe d'un signal non codé, c'est-à-dire d'un signal sinusoïdal de l'art antérieur,
"PrNC" puissance réfléchie d'un signal non codé, c'est-à-dire d'un signal sinusoïdal de l'art antérieur,
"PdC" puissance directe d'un signal codé, c'est-à-dire
   de type aléatoire ou pseudo-aléatoire de l'invention,
"PrC" puissance réfléchie d'un signal codé, c'est-à-dire de type aléatoire ou pseudo-aléatoire de l'invention.

Les mesures sont effectuées à différentes puissances d'une part avec un signal non codé (NC) (signal monochromatique à 1,11 MHz) ou sinusoïdal continu de type de l'art antérieur, tel que le signal S₁ des figures 1 et 2, qui permet d'obtenir la courbe 1 avec les cercles ou les points de la figure 6, à titre de comparaison.

D'autre part, ce même signal modulé par un code pseudo-aléatoire permet d'obtenir un signal électronique pseudo-aléatoire de séquence M selon la présente invention tel que représenté aux figures 3 et 4 référencé AS, qui permet d'obtenir la courbe 2 avec les losanges de la figure 6.

Dans les deux cas, le signal généré est maintenu jusqu'à l'obtention d'une valeur plateau de luminescence. L'injection de la solution dans la cuve se fait manuellement avec une seringue de 50 ml et toujours de la même façon.

Pour chaque puissance testée, la mesure est répétée 4 fois avec la même solution. Entre ces quatre mesures, la solution est aspirée de la cuve de mesure puis réinjectée afin de garder un degré de saturation constant. Par contre, lors du passage à une puissance autre, la solution est jetée et renouvelée.

La température est relativement stable (23-28°C), le temps d'irradiation étant court et le transducteur étant seulement refroidi par l'arrière avec un circuit d'eau froide (non représenté) sur sa partie métallique.

Les résultats obtenus sont donnés dans le tableau I ci-après.

Vᵢₙ (mV) correspond à la tension d'entrée sur l'amplificateur de puissance 130 de type Prana. P_{d} et Pᵣ correspondent respectivement aux puissances directes et réfléchies lues sur le wattmètre 132, tandis que P_{Trans} est la puissance transmise par le transducteur (P_{Trans} = P_{d}-Pᵣ). Lum représente la valeur de luminescence lue sur le nanoampèremètre 186. Les indices C et NC caractérisent respectivement un signal codé de type aléatoire ou pseudo-aléatoire (A.S.) selon la présente invention (figures 3 et 4) et un signal non codé, c'est-à-dire sinusoïdal de type S1 selon l'art antérieur (figures 1 et 2).

Les valeurs de luminescence obtenues à partir d'un signal codé selon l'invention sont représentées par la courbe 2 avec des losanges à la figure 6, et avec un signal non codé sinusoïdal de l'art antérieur par la courbe 1 avec des cercles ou points à la figure 6, en fonction de la puissance du signal transmis par le transducteur exprimée en Watt. Pour des valeurs inférieures à 10 W, la luminescence mesurée pour les deux signaux correspond à la luminescence naturelle de l'eau.

Le seuil de cavitation est obtenu autour de 12 à 15 W pour le signal non codé, de type sinusoïdal continu de l'art antérieur alors qu'il n'est jamais atteint dans la gamme de puissance utilisée avec le signal codé de type aléatoire ou pseudo-aléatoire selon la présente invention. Il n'a pas été possible de faire des mesures au-delà de 40 W dans mettre en danger le transducteur ultrasonore 123.

| Vin (mV) | PdNc (W) | PrNc (W) | PdC (W | PrC (W) | LumNC (nA) | LumC (nA) | P_{Trans}NC (W) | P_{Trans}C (W) |
|---|---|---|---|---|---|---|---|---|
| 34 | 13,0 | 0,6 | 31,5 | 19,0 | 29 | 49 | 12,4 | 12,5 |
| 39 | 15,3 | 0,6 | 39,5 | 24,1 | 130 | 61 | 14,7 | 15,4 |
| 44 | 20,0 | 0,9 | 50,8 | 31,1 | 600 | 71 | 19,1 | 9,7 |
| 49 | 26,3 | 1,5 | 61,2 | 37,2 | 1400 | 78 | 24,8 | 24,0 |
| 55 | 32,1 | 1,7 | 77,8 | 47,8 | 2600 | 95 | 30,4 | 30,0 |
| 62 | 41,0 | 2,2 | 98,3 | 59,6 | 4325 | 89 | 38,8 | 38,7 |

Ainsi, on constate de manière inattendue qu'avec un signal électronique large bande selon la présente invention, et par exemple un signal électronique codé de type pseudo-aléatoire ou aléatoire, aucun phénomène de cavitation n'apparaît dans la gamme de puissance utilisée pour l'expérience, ce qui se traduit par l'obtention d'une courbe de luminescence constante en fonction de la puissance, contrairement à la courbe 1 obtenue avec un signal sinusoïdal conforme à l'art antérieur.

Ceci constitue un résultat surprenant de la présente invention.

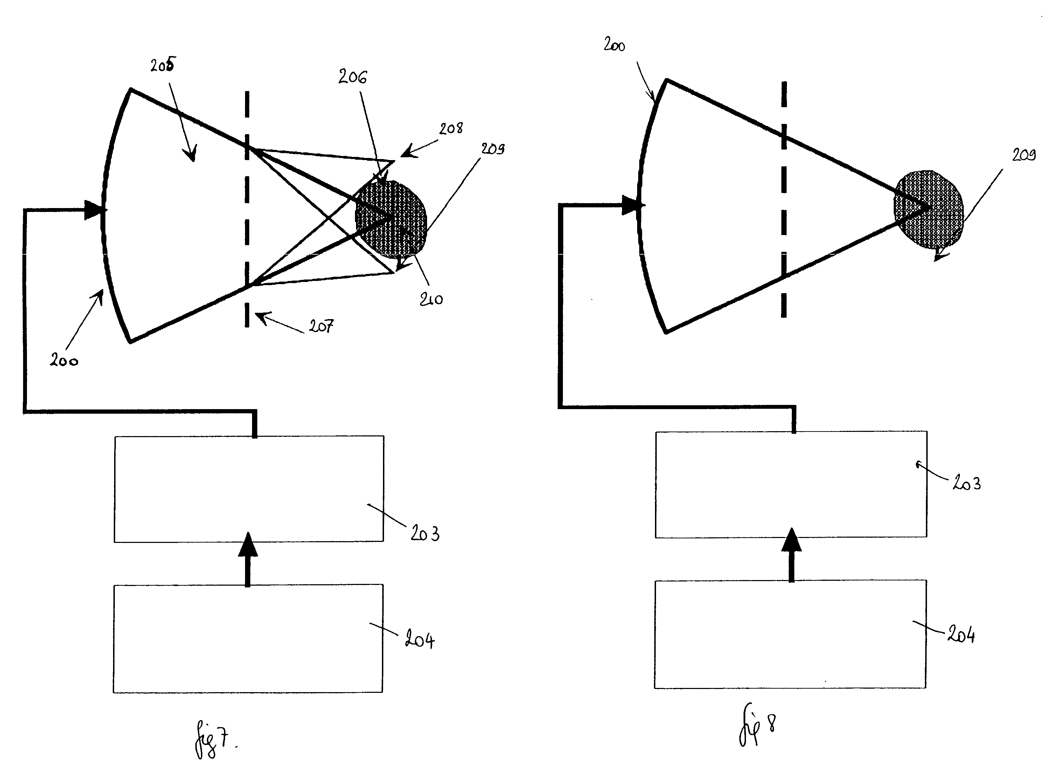

La figure 7 représente un schéma de principe du fonctionnement d'un dispositif de l'art antérieur, en présence d'une structure périodique ou quasi-périodique; apparaît sur la figure 7 un dispositif de thérapie 200, relié via un amplificateur 203 à un générateur de signal 204.

Comme décrit plus haut, le dispositif de thérapie est par exemple une calotte sphérique à focalisation naturelle, qui comporte un ou plusieurs éléments transducteurs (mono-transducteur, ensemble de céramiques annulaires, mosaïque).

Le dispositif de thérapie crée un champ ultrasonore 205 dirigé vers la zone à traiter 206. Une structure 207 périodique ou quasi-périodique est disposée dans le champ ultrasonore 205, entre le dispositif de thérapie 200 et la zone à traiter 206, avec un point focal 210. Cette structure peut, par exemple, être constituée, dans le cas du traitement des tumeurs du foie par voie externe, par le gril costal.

Dans les dispositifs de l'art antérieur, excités par un signal classique de type sinusoïdal, ou plus généralement excités par des signaux périodiques, une telle structure périodique provoque l'apparition de points de focaux secondaires 208, 209 (ou lobes secondaires), en dehors de la zone à traiter, par diffraction des ondes ultrasonores à travers la structure 207. L'apparition de ces points de focalisation secondaires a des conséquences évidentes sur la sécurité du traitement. De plus, le rayonnement d'énergie vers ces points secondaires diminue l'intensité rayonnée vers le point focal 210 et peut compromettre l'efficacité du traitement. On peut dire que dans cette configuration, des phénomènes de focalisation secondaires se produisent derrière la structure périodique ou quasi-périodique. On utilise l'expression "derrière la structure 207", pour signifier "en aval de la structure 207, sur le trajet de propagation des ondes ultrasonores".

La figure 8 représente un schéma analogue à celui de la figure 7, dans un dispositif mettant en oeuvre le dispositif de thérapie, un générateur de signal délivrant un signal électronique large bande. Comme décrit ci-dessus, le générateur de signal peut délivrer un signal électronique aléatoire ou pseudo-aléatoire, et plus particulièrement un signal électronique pseudo-aléatoire codé de Golay ou de Barker, ou encore un signal électronique codé de type pseudo-aléatoire de séquence M (par exemple du type décrit plus haut).

On peut aussi caractériser l'invention en ce que le dispositif de thérapie est excité par un signal présentant une fonction d'autocorrélation aussi proche que possible d'un Dirac. Les signaux visés ci-dessus ont une fonction d'auto-corrélation qui s'approche d'un Dirac.

Les résultats de l'invention sont représentés sur la figure 8; les points de focalisation secondaires provoqués par la structure périodique ou quasi-périodique ont disparu; en effet, la position de ces points focaux dépend essentiellement de la périodicité de la structure 207 et de la fréquence des ultrasons. L'utilisation de signaux large bande fait varier au cours du traitement la position des points de focalisation secondaires. De la sorte, il n'y a plus d'accumulation locale d'énergie ailleurs que dans la zone à traiter, au voisinage du point de focalisation principal 210. L'invention permet donc de diminuer le risque de lésions secondaires en dehors de la zone à traiter, même en présence d'une structure périodique ou quasi-périodique.

L'invention rend possible un traitement précis et localisé, même derrière une structure périodique ou quasi-périodique, et par exemple un traitement du foie derrière le gril costal.

Les divers modes de réalisation de l'invention, décrits en référence aux figures 7 et 8 et en référence aux figures précédentes, peuvent évidemment être combinés.

Par ailleurs, les figures 3 à 8 font partie intégrante de la présente invention et donc de la présente description.

## Revendications

1. Procédé de génération d'ondes ultrasonores dans un milieu de propagation, comprenant l'activation d'au moins un élément transducteur ultrasonore par un signal électronique délivré par un générateur de signal, **caractérisé en ce qu**'on réduit ou empêche les phénomènes de cavitation résultant de la propagation des ondes ultrasonores émises par l'élément de transducteur ultrasonore dans le milieu de propagation en utilisant un générateur de signal délivrant un signal électronique activant l'élément transducteur pour émettre un signal acoustique large bande, dont la largeur de bande vaut environ 50% de la fréquence centrale du générateur de signal.

2. Procédé selon la revendication 1, **caractérisé en ce qu**'on utilise un général de signal délivrant un signal électronique aléatoire ou pseudo-aléatoire.

3. Procédé selon la revendication 1, **caractérisé en ce que** le générateur de signal précité délivre un signal électronique pseudo-aléatoire codé de Golay.

4. Procédé selon la revendication 1, **caractérisé en ce que** le générateur de signal précité délivre un signal électronique pseudo-aléatoire codé de Barker.

5. Procédé selon la revendication 1 ou 3, **caractérisé en ce que** le générateur de signal précité délivre un signal électronique pseudo-aléatoire codé de séquence M, de préférence ayant une durée d'impulsion élémentaire thêca comprise entre 0,1 *µ*s et 100 *µ*s, idéalement 1 *µ*s, une période de répétition T comprise entre 1 *µ*s et 10 s, idéalement comprise entre 0,5 et 5 s.

6. Procédé selon la revendication 1, **caractérisé en ce que** ledit signal électronique présente une fonction d'autocorrélation s'approchant d'un Dirac.

7. Appareil de thérapie comprenant un dispositif de thérapie (120) proprement dit comprenant au moins un élément transducteur ultrasonore de thérapie (123) et un générateur de signal (140) délivrant un signal électronique audit élément transducteur ultrasonore, **caractérisé en ce que** le générateur de signal (140) délivre un signal électronique aléatoire ou pseudo-aléatoire (A.S.) activant l'élément transducteur pour émettre un signal acoustique large bande, dont la largeur de bande vaut environ 50% de la fréquence centrale du générateur de signal.

8. Appareil selon la revendication 7, **caractérisé en ce que** le générateur de signal (140) délivre un signal aléatoire Gaussien ou Poissonien.

9. Appareil selon la revendication 7, **caractérisé en ce que** le générateur de signal (140) délivre un signal pseudo-aléatoire codé à séquence M, de fréquence présentan durée d'impulsion élémentaire thêta comprise entre 0,1 *µ*s et 100 *µ*s, idéalement d'environ 1 *µ*s, à une période de répétition T comprise entre 1 *µ*s et 10 s, idéalement comprise entre 0,5 et 5 s.

10. Appareil selon la revendication 7, **caractérisé en ce que** le générateur de signal (140) précité délivre un signal pseudo-aléatoire codé de Golay, ou un signal pseudo-aléatoire codé de Barker.

11. Appareil selon l'une des revendications 7 à 10, **caractérisé en ce que** l'élément transducteur ultrasonore (123) est un élément transducteur qui génère des ondes ultrasonores lorsqu'il est excité par un signal dont la fréquence est sensiblement différente de sa fréquence nominale.

12. Appareil selon l'une des revendications 7 à 11, **caractérisé en ce que** l'élément transducteur ultrasonore (123) est choisi parmi le groupe consistant en un monoélément, un réseau annulaire cu une mosaïque d'éléments transducteurs ultrasonores, de préférence l'élément transducteur ultrasonore de thérapie étant du type focalisé, en particulier à focalisation géométrique, par exemple sous forme de coupelle sphérique.

13. Procédé de génération d'ondes ultrasonores dans un milieu de propagation, comprenant l'activation d'au moins un élément transducteur ultrasonore (200) par un signal électronique périodique délivré par un générateur de signal (204), **caractérisé en ce que** l'on réduit ou l'on empêche les phénomènes de focalisation secondaire derrière une structure périodique ou quasi-périodique (207) en utilisant un générateur de signal délivrant un signal électronique activant l'élément transducteur pour émettre un signal acoustique large bande, dont la largeur de bande vaut environ 50% de la fréquence centrale du générateur de signal.

14. Procédé selon la revendication 13, **caractérisé en ce que** ledit signal électronique est un signal aléatoire ou pseudo-aléatoire.

15. Procédé selon la revendication 13, **caractérisé en ce que** ledit signal électronique est un signal aléatoire ou pseudo-aléatoire Gaussien ou Poissonien.

16. Procédé selon la revendication 13, **caractérisé en ce que** ledit signal électronique est un signal pseudo-aléatoire codé de Barker ou codé de Golay.

17. Procédé selon la revendication 13, **caractérisé en ce que** ledit signal électronique est un signal pseudo-aléatoire codé à séquence M, de fréquence présentant une durée d'impulsion élémentaire thêta comprise entre 0,1 *µ*s et 100 *µ*s, idéalement d'environ 1 *µ*s, et une période de répétition T comprise entre 1 *µ*s et 10 s, idéalement comprise entre 0,5 et 5 s.

18. Procédé selon la revendication 13, **caractérisé en ce que** ledit signal électronique présente une fonction d'autocorrélation s'approchant d'un Dirac.

## Claims

1. A method for generating ultrasound waves in a propagation medium comprising activating at least one ultrasound transducer element by an electronic signal supplied by a signal generator characterized by reducing or preventing cavitation phenomena resulting from the propagation of ultrasound waves emitted by the ultrasound transducer element within the propagation medium by the use of a signal generator activating the transducer element to supply a wideband acoustic signal with a bandwidth equal to about 50% of the central frequency of the signal generator.

2. A method according to claim 1, **characterized in that** said signal generator is a signal generator supplying a random or pseudo-random electronic signal.

3. A method according to claim 1, **characterized in that** said signal generator supplies a Golay coded pseudo-random electronic signal.

4. A method according to claim 1, **characterized in that** said signal generator supplies a Barker coded pseudo-random electronic signal.

5. A method according to claim 1 or 3, **characterized in that** said signal generator supplies an M-sequence pseudo-random type coded electronic signal, preferably having an elementary pulse duration comprised between 0.1 *µ*s and 100 *µ*s, ideally of 1 *µ*s and a period of repetition T comprised between 1 *µ*s and 10 s, and ideally of between 0.5 and 5 s.

6. The method according to claim 1, **characterized in that** said electronic wideband signal has an autocorrelation function approaching a Dirac function.

7. Therapy apparatus comprising an actual therapy device (120) comprising at least one ultrasound therapy transducer element (123) and a signal generator (140) supplying an electronic signal to said ultrasound transducer element, **characterized in that** said signal generator (140) supplies a wideband electronic signal of the random or pseudo-random type (A.S.) activating the transducer element to supply a wideband acoustic signal with a bandwidth equal to about 50% of the central frequency of the signal generator.

8. The therapy apparatus according to claim 7, **characterized in that** said signal generator (140) supplies a Gaussian or Poissonian distribution type random signal.

9. The therapy apparatus according to claim 7, **characterized in that** said signal generator (140) supplies an M-sequence pseudo-random type coded electronic signal, with an elementary pulse duration theta comprised between 0.1 *µ*s and 100 *µ*s and ideally of about 1 *µ*s, and a period of repetition T comprised between 1 *µ*s and 10 s and ideally comprised between 0.5s and 5 s.

10. The therapy apparatus according to claim 7, **characterized in that** said signal generator (140) supplies a Golay coded or Barker coded pseudo-random signal.

11. The therapy apparatus according to one of claims 7 to 10, **characterized in that** said ultrasound transducer element (123) is a wideband transducer element for generating ultrasound waves when excited by a signal the frequency of which is substantially different from its nominal frequency.

12. The therapy apparatus according to one of claims 7 to 11, **characterized in that** said ultrasound transducer element (123) is selected from the group comprising a single element, an annular array or a mosaic of ultrasound transducer elements, said therapy ultrasound transducer element being preferably of the focused type, particularly using geometrical focusing, and for example being in the form of a semi-spherical cup.

13. A method for generating ultrasound waves in a propagation medium comprising activating at least one ultrasound transducer element (200) by an electronic signal supplied by a signal generator (204), **characterized in that** secondary focusing phenomena behind a periodic or quasi-periodic structure (207) is reduced or prevented by the use of a signal generator activating the transducer element to supply a wideband acoustic signal with a bandwidth equal to about 50% of the central frequency of the signal generator.

14. A method according to claim 13, **characterized in that** said electronic signal is a random or pseudo-random signal.

15. The method according to claim 13, **characterized in that** said electronic signal is a Gaussian or Poissonian distribution type random signal.

16. The method according to claim 13, **characterized in that** said wideband electronic signal is a Barker coded or Golay coded pseudo-random signal.

17. The method according to claim 13, **characterized in that** said electronic signal is a M-sequence pseudo-random type coded electronic signal of a frequency having an elementary pulse duration comprised between 0.1 *µ*s and 100 *µ*s and ideally of about 1 *µ*s, and a period of repetition T comprised between 1 *µ*s and 10 s and ideally comprised between 0.5s and 5 s.

18. The method according to claim 13 **characterized in that** said electronic wideband signal has an autocorrelation function approaching a Dirac function.

## Patentansprüche

1. Verfahren zur Erzeugung von Ultraschallwellen in einem Ausbreitungsmedium, bestehend aus der Aktivierung zumindest eines Ultraschallwandlerelementes durch ein von einem Signalgeber gegebenes elektronisches Signal, **dadurch gekennzeichnet**, dass Kavitationserscheinungen, welche aus der Ausbreitung der durch den Wandler in das Ausbreitungsmedium gesendeten Ultraschallwellen resultieren, reduziert bzw. gehemmt werden, indem ein Signalgeber verwendet wird, der ein elektronisches Signal sendet, das den Wandler zur Abgabe eines akustischen Breitbandsignals aktiviert, dessen Bandbreite der Frequenz ungefähr 50% der mittleren Frequenz des Signalgebers benötigt.

2. Verfahren nach Anspruch 1, gekennzeichnet durch die Verwendung eines Signalgebers, der ein zufälliges bzw. quasi-zufälliges elektronisches Signal sendet.

3. Verfahren nach Anspruch 1, **dadurch gekennzeichnet**, dass der vorgenannte Signalgeber ein quasi-zufälliges elektronisches Signal mit der Kodierung 'Golay' sendet.

4. Verfahren nach Anspruch 1, **dadurch gekennzeichnet**, dass der vorgenannte Signalgeber ein quasi-zufälliges elektronisches Signal mit der Kodierung 'Barker' sendet.

5. Verfahren nach Anspruch 1 oder 3, **dadurch gekennzeichnet**, dass der vorgenannte Signalgeber ein quasi-zufälliges elektronisches Signal mit der Kodierung 'Sequenz M' sendet, mit einer elementaren Impulsdauer Theta von zwischen 0,1 *µ*s und 100 *µ*s, idealerweise 1 *µ*s, einer Wiederholungszeit T von zwischen 1 *µ*s und 10 s, idealerweise zwischen 0,5 und 5 s.

6. Verfahren nach Anspruch 1, **dadurch gekennzeichnet**, dass das elektronische Signal eine sich an eine Dirac[-Funktion] annähernde Autokorrelationsfunktion darstellt.

7. Therapeutischer Apparat, bestehend aus einer therapeutischen Vorrichtung (12), welche im eigentlichen Sinne aus zumindest einem therapeutischen Ultraschallwandlerelement (123) und einem Signalgeber (140) besteht, der ein elektronisches Signal an den Ultraschallwandler sendet, **dadurch gekennzeichnet**, dass der Signalgeber (140) ein zufälliges oder quasi-zufälliges (A.S.) elektronisches Signal sendet, wodurch der Ultraschallwandler zur Abgabe eines akustischen Breitbandsignals aktiviert wird, dessen Bandbreite der Frequenz ungefähr 50% der mittleren Frequenz des Signalgebers benötigt.

8. Apparat nach Anspruch 7, **dadurch gekennzeichnet**, dass der Signalgeber (140) ein zufälliges Signal nach Gauß oder Poisson abgibt.

9. Apparat nach Anspruch 7, **dadurch gekennzeichnet**, dass der Signalgeber (140) ein quasi-zufälliges Signal mit der Kodierung 'Sequenz M' und einer Frequenz abgibt, die eine elementare Impulsdauer Theta von zwischen 0,1 *µ*s und 100 *µ*s, idealerweise ungefähr 1 *µ*s, bei einer Wiederholungszeit T von zwischen 1 *µ*s und 10 s, idealerweise zwischen 0,5 und 5 s, darstellt.

10. Apparat nach Anspruch 7, **dadurch gekennzeichnet**, dass der Signalgeber (140) ein quasi-zufälliges Signal mit der Kodierung 'Golay' oder ein quasi-zufälliges Signal mit der Kodierung 'Barker' abgibt.

11. Apparat nach einem der Ansprüche 7 bis 10, **dadurch gekennzeichnet**, dass das Ultraschallwandlerelement (123) ein Wandler ist, der Ultraschallwellen erzeugt, wenn er durch ein Signal angeregt wird, dessen Frequenz sich von seiner Nennfrequenz deutlich unterscheidet.

12. Apparat nach einem der Ansprüche 7 bis 11, **dadurch gekennzeichnet**, dass das Ultraschallwandlerelement (123) aus der Gruppe, bestehend aus einem Monoelement, einem Ringnetz oder einem Mosaik aus Ultraschallwandlern, ausgewählt wird, wobei das therapeutische Ultraschallwandlerelement vorzugsweise mit einer Fokussierung ausgeführt ist, insbesondere einer geometrischen Fokussierung, beispielsweise in Form einer kugelförmigen Schale.

13. Verfahren zur Erzeugung von Ultraschallwellen in einem Ausbreitungsmedium, bestehend aus der Aktivierung zumindest eines Ultraschallwandlerelementes (200) durch ein von einem Signalgeber (204) abgegebenes periodisches elektronisches Signal, **dadurch gekennzeichnet**, dass die sekundären Fokussierungserscheinungen hinter einer periodischen oder quasi-periodischen Struktur (207) reduziert oder gehemmt werden, indem ein Signalgeber verwendet wird, der ein elektronisches Signal sendet, welches das Ultraschallwandlerelement zur Abgabe eines akustischen Breitbandsignals aktiviert, dessen Bandbreite der Frequenz ungefähr 50% der mittleren Frequenz des Signalgebers benötigt.

14. Verfahren nach Anspruch 13, **dadurch gekennzeichnet**, dass das elektronische Signal ein zufälliges oder quasi-zufälliges Signal ist.

15. Verfahren nach Anspruch 13, **dadurch gekennzeichnet**, dass das elektronische Signal ein zufälliges oder quasi-zufälliges Signal nach Gauß oder Poisson ist.

16. Verfahren nach Anspruch 13, **dadurch gekennzeichnet**, dass das elektronische Signal ein quasi-zufälliges Signal mit der Kodierung 'Barker' oder der Kodierung 'Golay' ist.

17. Verfahren nach Anspruch 13, **dadurch gekennzeichnet**, dass das elektronische Signal ein quasi-zufälliges Signal mit der Kodierung 'Sequenz M' und einer Frequenz ist, die eine elementare Impulsdauer Theta von zwischen 0,1 *µ*s und 100 *µ*s, idealerweise ungefähr 1 *µ*s, und eine Wiederholungszeit T von zwischen 1 *µ*s und 10 s, idealerweise zwischen 0,5 und 5 s, darstellt.

18. Verfahren nach Anspruch 13, **dadurch gekennzeichnet**, dass das elektronische Signal eine sich an eine Dirac [-Funktion] annähernde Autokorrelationsfunktion darstellt.
